# EUROPEAN PATENT APPLICATION

(11) **EP 3 067 060 A1**
(43) Date of publication of application: **14.09.2016**
(21) Application number: 14880930.4
(22) Date of filing: 29.12.2014
(51) Int. Cl.: A61K 36/886, A61K 8/97, A61P 17/02, A61Q 17/04

(54) **COLD NATURAL ALOE VERA GEL WITHOUT STABILISERS**

(30) Priority: 30.01.2014 ES 201430109
(71) Applicant: Tello De Juan, Maria Del Mar, 41219 Las Pajanosas (Sevilla) (ES)
(72) Inventor: Tello De Juan, Maria Del Mar, 41219 Las Pajanosas (Sevilla) (ES)
(74) Representative: Bartrina Diaz, José María
(86) International application number: PCT/ES2014/070986
(87) International publication number: WO 2015/114181

(57) **Abstract**

This invention refers to a procedure that makes it possible to use a pure aloe vera gel which contains all the natural properties of the plant, based on the incorporation of a stabilisation process achieved through freezing, whereby it is possible to topically apply the cold cream, lotion, pomade, or ointment, achieving all the typical uses of Aloe Vera, though primarily to alleviate all kinds of burns.

## Description

### OBJECT OF THE INVENTION

This invention, as expressed in the statement of this descriptive report, refers to a procedure based on which it is possible to use a pure aloe vera gel which contains all the natural properties of the plant, based on the incorporation of a stabilisation process achieved through freezing.

### BACKGROUND TO THE STATE OF THE ART

Aloe is a genus of the subfamily Asphodeloideae, belonging to the Liliaceae family, which includes over 200 species, but only one of them is vera.

Thanks to its healing properties, the use of aloe goes back to humanity's origins. Even as early as the first century of our era, Dioscorides described it in detail in his Greek herbarium, highlighting its medicinal and cosmetic properties.

Today we know of the benefits it has for our skin, given that it regenerates our fibroblast cells (cells of the connective tissue in our skin) between 6 and 8 times faster than normal cellular reproduction alone. It also nourishes our skin with over 20 vitamins, amino acids, minerals, polysaccharides, anthraquinones, enzymes, etc., and it removes dead cells and regulates pH levels. Thus, their use in cosmetics especially designed to treat the skin is well known: it can remove or alleviate wrinkles, spots on the skin, eczemas, scratches, cellulite, stretch marks, scars, herpes, haemorrhoids, insect bites, itching, psoriasis, all kinds of burns, etc.

From a pharmacological point of view, the active ingredient is comprised by the desiccated juice of the secreting cells of the leaves. The smell is distinctive and strong, while the taste is bitter and unpleasant. Two compounds can be obtained from the leaves:
a) Gel, which is the mucilaginous part of the mesophilic or issue parenchyma in the centre of the leaves. Plants which are more exposed to the sun make less pulp and more latex. A bright and bitter gel is extracted from the pulp, which in turn is obtained by extruding the inside of the leaves. First, all of the anthraquinone content must be removed from the epidermis of the leaves. If this process is not carried out, the latex becomes oxidised and becomes brown easily. The fragility of some of the components of the gel means that it is necessary to stabilise the recently obtained material and preserve it against bacterial contamination.
b) Aloe or latex is the set juice resulting from cutting the leaves; it is a very bitter brown crystalline solid, known as aloe juice. It is found in the pericyclic cells near the ducts just under the epidermis, between the mucilaginous and chlorophyllic parenchyma. It is usually obtained by allowing the fluid that comes out of cut leaves to flow, and depositing said fluid in a container mixed with the pulp.

In order to prevent the loss of latex, the leaves must be cut near the base, close to the stem. It should be taken into account that any leaf that is cut will not grow again. In order to use it with the skin, it is cut along the middle, or if the aim is to only extract the latex, the skin is removed beforehand. Once removed, the leaves are washed and filleted. The skin and yellowish coat (allantoin) are separated.

Regarding the current state of the art on this matter, there are currently many cosmetic products on the market whose main ingredient is aloe vera, but it is always stabilised (mixed with other ingredients, be they natural or artificial, to help preserve them; or they are subjected to a heating process to solidify it and use it as a powder). This is because aloe vera naturally oxidises quickly when it comes into contact with the air at temperatures greater than 8ºC, therefore losing all of its properties in a matter of hours.

There are numerous inventions that use Aloe Vera as a cosmetic product or to treat skin in general.

Thus, in the patent with publication number and title ES2232303B1, "Composición de crema cosmética y/o terapéutica" ["Composition of cosmetic and/or therapeutic cream"] respectively, which has a 44.8% weight content of Aloe vera, 44.8% weight content of virgin olive oil, and 10.4% virgin beeswax, natural products are used to create a cream with cosmetic and/or therapeutic applications for the skin, including: burns, cuts, skin infections, allergies, psoriasis, rashes, varicose veins, herpes, sores, and other issues which may arise on the skin.

In addition, Spanish patent 2123465 protects a dermatological composition which regenerates hair and is used to protect the skin, and which contains demineralised water, alcohol, and vegetable extracts from thyme, lemon, Aloe, and others, as well as a small amount of vitamin B6.

In European patent 0425398 regarding a procedure for preparing an "aloe water" which includes the condensation of the vapour created by boiling crushed aloe leaves, the condensation liquid or aloe water is used to prepare cosmetic products aimed at treating the skin or hair.

Lastly, international patent W9526198 proposes "Metal complexes of aloe extracts for skin and hair care".

All of these are effective for skin treatment, making use of Aloe vera to different degrees as the fundamental element in their composition, or as the element which provides the benefits of application; however, the method by which it is obtained and preserved until it can be used by the end user forces the use of external stabilising agents.

However, the "Natural Aloe Vera cold gel without stabilisers" akes a contribution to the state of the art with the application of Aloe Vera in cold form after some minutes have passed to allow for defrosting, or even in its frozen form if applied to burns, based on the incorporation of a stabilisation process through freezing which has the following advantages:
1. Greater efficiency of application or lower duration of treatment by accelerating the healing process.
2. Lower risk of side effects for the body, as it does not include any additional element in its composition.
3. Better penetration in the three layers of skin: epidermis, dermis, and hypodermis, compared to other cosmetics available on the market.

### DETAILED DESCRIPTION OF THE INVENTION

In order to explain the "Natural Aloe Vera cold gel without tabilisers", the procedure by which the product is obtained is outlined below:
1. Selection and cutting of basal, hard, fleshy, and healthy leaves of adult plants at least 4 years old,
2. Washing in cold water, rubbing them by hand until all impurities and traces of dirt which may be stuck to the leaves are removed.
3. Cutting each leaf with a knife or similar tool along its base from one side to the other, removing between two and three centimetres of flesh on each leaf, and placing the leaf vertically for three hours to let the aloin run out.
4. Second washing of the leaves to remove the rest of the aloin.
5. The next step is the extraction of the gelatin inside each leaf. To do so, two centimetres are cut off the base of the leaf again to remove the part contaminated by the air and the brown/yellow aloin.
6. Next, the tip and dented sides of each leaf are cut, and then the green skin around the leaf is removed with a knife, and the gelatin is extracted from the leaf.
7. The gelatin is used to obtain the aloe vera gel, which is characterised by its mucilaginous appearance, its dense, sticky texture, and clear colour. To do so, a crushing machine or liquidiser is used, manufactured using stainless steel and plastic components, fitted with a filter or sieve, and taking into account that this process has to be carried out without raising the temperature of the product above 8º C at any given time.

Once the gelatin has been liquidised and sieved, the gel is obtained, which is stored in individual PET or similar plastic containers, designed to withstand temperatures as low as -19ºC without suffering any damage.

Based on the procedure described above, the first aloe vera gel is obtained and frozen at a temperature between -5 and -19º C, which serves as the stabilisation mechanism, preventing any of the properties from being lost until the time of consumption on the market as a natural cosmetic.

The table below indicates how analytical laboratory reports show that after being frozen for three months, the properties of the cold Aloe vera gel have not been affected; that is, they are in the same condition as if they had been made that same day, with no oxidation, deterioration, or any other kind of contamination.

| DETERMINATION | RESULTS |
|---|---|
| Total aerobic count 37º C | 0 utc/cc |
| Total anaerobic count 37º C | 1 utc/cc |
| Mould | 0 utc/cc |
| Yeasts | 0 utc/cc |

The use of the Aloe vera gel described in this document is illustrated with the following examples:

### Example 1

People who have jobs where their eyes are at risk of potential burning of the conjunctiva due to sparks, such as welders, are aware of the discomfort which these kinds of burns can cause. The clear gelatine from fresh Aloe vera leaves is sterile and soothing. Therefore, one or two direct applications in the eye or eyes or the individuals affected can quickly alleviate all the symptoms and speed up the healing process. In fact, conjunctivitis can be sever or chronic, and in both cases Aloe Vera is suitable for treatment either as drops or as a gelatin.

Using the cold Aloe Vera gel without stabilisers in these cases would eliminate the need to cut a leaf from the plant, which would waste a whole leaf when only a few drops of gel are needed each day to treat conjunctivitis, or for any other cosmetic or therapeutic application used on a daily basis. With this invention, simply by defrosting, in this case, a single dose of 1.5 ml, there is enough gel for daily treatment.

### Example 2

A 55-year-old woman accidentally spills a pot of boiling soup on herself in the kitchen, suffering third degree burns on her legs and feet. Thanks to the quick reaction of her daughter, who heard her screams and poured cold water on her legs, and then dressed her legs with frozen aloe vera leaf gels, alleviating the pain until the gelatins defrosted and the gel ran out, after one week of treatment based on frozen aloe vera gelatin and gel, the burns healed without leaving any scars.

Natural Aloe Vera cold gel without stabilisers can be stored in any home, frozen in suitable doses to alleviate the heat which builds up in the skill after burns of all kinds, rapidly cooling burned skin, and the frozen gel neutralises the pain while it melts and is absorbed by the skin.

The clinical results given above clearly show that the Aloe vera gel which is the object of this invention is useful for the applications described.

## Claims

1. Natural Aloe Vera cold gel without stabilisers, **characterised by** the procedure used to obtain it, according to the following stages:
a) Selection and cutting of basal, hard, fleshy, and healthy leaves of adult plants at least 4 years old,
b) Washing in cold water, rubbing them by hand until all impurities and traces of dirt which may be on the leaves are removed.
c) Cutting each leaf with a knife or similar tool along its base from one side to the other, removing between two and three centimetres of flesh on each leaf, and placing the leaf vertically for three hours to let the aloin run out.
d) Second washing of the leaves to remove the rest of the aloin.
e) Extraction of the gelatin inside each leaf. To do so, two centimetres are cut off the base of the leaf again to remove the part contaminated by the air and the brown/yellow aloin.
f) Next, the tip and dented sides of each leaf are cut, and then the green skin around the leaf is removed with a knife, and the gelatin is extracted from the leaf.
g) The gelatin is crushed and sieved until obtaining the Aloe Vera gel, which is **characterised by** its mucilaginous appearance, its dense, sticky texture, and clear colour. To do this, a crushing machine or liquidiser are used, manufactured using stainless steel and plastic components, fitted with a filter or sieve, and taking into account that this process has to be carried out without raising the temperature of the product above 8º C at any given time.

2. Natural Aloe Vera cold gel without stabilisers according to the previous claim, characterised because the Aloe Vera gel obtained is stored and preserved frozen in individual PET or similar plastic containers, designed to withstand temperatures as low as -19ºC without suffering any damage.

3. Use of natural Aloe Vera cold gel without stabilisers according to claims 1 and 2, to be applied topically in the form of a cold cream, lotion, pomade, or ointment, for soothing serious or minor burns on the skin, be it thermal burns, sunburns, burns from UV rays from other sources, electrical burns, burns resulting from radiotherapy or laser surgery, or ocular burns.
